# EUROPEAN PATENT APPLICATION

(11) **EP 1 214 956 A2**
(43) Date of publication of application: **19.06.2002**
(21) Application number: 01500287.6
(22) Date of filing: 17.12.2001
(51) Int. Cl.: A61M 15/08

(54) **Nasal inhaler device**

(30) Priority: 18.12.2000 ES 200003091 U
(71) Applicant: Moreno Vidales, Benigno, 08004 Barcelona (ES)
(72) Inventor: Moreno Vidales, Benigno, 08004 Barcelona (ES)
(74) Representative: Carpintero Lopez, Francisco

(57) **Abstract**

The nasal inhaler device forms a physical base for an aromatic or therapeutic product that can be directly introduced into a person's nose so that it may carry out its action directly in the respiration, and that is made up of a laminate body (1) with a configuration in the shape of U that gives it a clip effect for attaching to the nasal septum, furthermore having two housings (2) at its ends dedicated to hold impregnators, infusers or porous capsules (3) that will contain the aromatic or therapeutic product in question.

## Description

### PURPOSE OF THE INVENTION

This invention refers to a nasal inhaler that allows the direct and personalized application of aromas and/or therapeutic products, directly in the user's nose.

Therefore, the purpose of the invention is to achieve a physical support for an aromatic or therapeutic product that may be directly introduced into a person's nose, so that it may act directly, in breathing, under optimum conditions.

### BACKGROUND TO THE INVENTION

In the aromatherapy environment diffusion systems are usually used that disperse the product in question into the atmosphere.

More specifically the product in question, conveniently housed in a container, is left in direct contact with the atmosphere that surrounds it, whether at ambient temperature or using a heat source, as a means of diffusion, ceramic, glass or metallic recipients being used, so that the gases generated from the product spread into the atmosphere, indirectly reaching people that occupy the space near them.

On occasions the aromatic product is integrated in the constituent wax of a candle, so that it emanates when its combustion takes place.

In this last case of application of heat to the aromatic product container, versions exist in which the product in question receives the heat from an under burner, as, for example, an alcohol burner, and others in which electrical heating is used, as, for example, heating the wick through which the product rises, by capillary action, from the inside of its recipient container to the exterior.

Regarding the products used, they can be solid and volatile or liquid, and in this latter case a spongy substance acting as a base, is soaked.

Also well known are aeration systems based on the on spray products, with gas propulsion or by mechanical pressure.

All these solutions are used both in aesthetic applications, as in perfumery, as in prophylactic-therapeutic applications, as in the case of anti-tobacco aromas or in the specific case of aromatherapy.

All these have as common denominator the fact that the product in question spreads in a greater or lesser space and arrives indirectly at the nasal passages of people located in the dwelling in question, which implies a double-sided difficulty, on one hand its effect is notably diminished at the level of the nasal passages by the dispersion in the air, and on the other hand, especially in the case of health products, the effect on people is lost when they leave the zone where the diffuser is.

On the other hand, as examples of devices that are inserted in the nasal cavities we could mention the Spanish Utility models numbers U 8801528, U 9701215, U 8803027 and U 8902241 that show different items used to improve breathing, as well as the American documents US 4887597, US 4120299 and US 4267831 that list items that are also inserted into the nasal cavities for different purposes, including that of administering medication as in the case of the US 4267831 and that in no case present the characteristics outlined in the invention that is described in this document.

### DESCRIPTION OF THE INVENTION

The inhaler device that the invention proposes solves fully and satisfactorily the previously set out difficulties since that it allows the personalized application of the product in question, specifically in the user's own nasal passages.

For this, and more specifically, the device is made up of a support that forms a kind of a clip, elastically deformable, attachable, using light pressure, to the user's nasal septum, this clip in turn made up of a laminate body, preferably of plastic, with an approximately omega profile, with the distinction that its side arms, at their ends, are bent contrary to the clip, forming in turn two small pincers or supplementary fastenings, in which impregnators, infusers or porous capsules, as for example in each of them cotton buds or of any other spongy or porous material, able to form the physical support for the aromatic and/or therapeutic product in question, are respectively placed.

This way, in each breathing cycle and during the inhalation, the jet of air that crosses the nasal orifices causes the partially picking-up of the product that impregnates these infusers or porous capsules, entering directly into the user's breathing passages, in a completely personalized way.

Obviously, the device can be used as a prosthesis in the case of direct medication for olfactory or other deficiencies, that the specialists can identify, or for neutralization of allergies and clinical patterns pertaining to medical fields that may be treated in this way and by this dosage system.

Although previously it has been said that the body of the device forms a clip for attachment to the user's nasal septum, with a infuser in each of its ends, obviously the device can do without one of these infusers, when only the other one is necessary, or it can adopt any other attachment system to the user's nose.

### DESCRIPTION OF THE DRAWINGS

To supplement the description that is being carried out and with the purpose of helping to a better understanding of the characteristics of the invention, in accordance with a preferable example of its practical operation, it is accompanied by a set of drawings in which illustratively and not limiting, the following has been depicted:
Figure 1. - Shows, in a perspective view, a nasal inhaler device made in accordance with the purpose of this invention.
Figure 2. - Shows, in an outline representation of elevation cross-section, the device of the previous figure properly attached to the nose of a user.

### PREFERABLE EXECUTION OF THE INVENTION

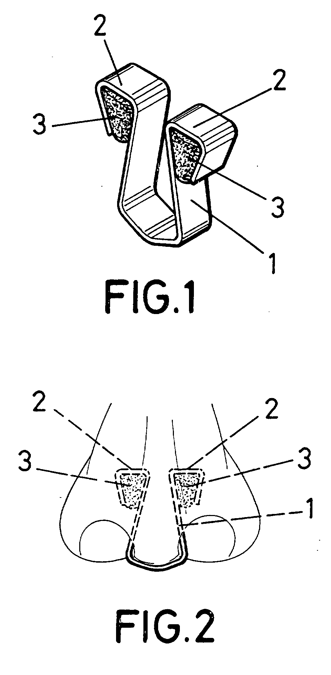

In view of the figures indicated, and specifically Figure 1, it can be seen how the nasal inhaler device that the invention proposes is formed from a laminate body (1), preferably of semi-rigid and elastic plastic, although equally it could be metallic or of any other material that gives this body a clip effect, thanks to it having a basic "U" configuration, that allows its joining and securing to the user's nasal septum, the end of the side arms of this body extending to form a kind of omega, and specifically two housings or secondary pincers (2) in its ends, dedicated to holding and securing the respective impregnators, infusers or porous capsules (3) that as has been said previously can be made up of a small cotton bud or of any other spongy or porous material, able to retain in its interior the aromatic or therapeutic product in question, a product that, as has also been said previously, will be carried by the air to the user's breathing passages by his own natural breathing.

Obviously this configuration that appears in the drawings for the laminate body (1), both as refers to its central and securing to the nose area as at its ends for attachment of the infusers (3), is merely exemplary, being variable practically without more limitation than that set by its necessity for attachment to the nasal septum, by its action as support for the infusers and by the variations that its manufacture and distribution may advise such as could be, for example, the incorporation of some central grooves or similar to fold in use.

In this sense it is also feasible that the body (1) be made up as a semi-ring of plastic, metal or any other appropriate material, that incorporates an infuser only in one of its ends, with a cross bracket instead of clip and that may be externally embedded in the nasal wings, or that the body support is made of a little housing externally attachable to the nose on the nasal wings and the bridge, or even in any aesthetic or similar adaptation such as "piercings" or any other practical execution that is considered desirable.

In any case it will be possible to introduce from source the aromatic or therapeutic product in question into the infuser, in which case it will be sold neatly stored inside a bag or plastic, aluminum or another tightly closed material recipient, or the product in question can, to start with, be physically independent of the device, the infusers of the latter being impregnated at an opportune moment, for example at first having the product in question inside a vial, flask or similar.

## Claims

1. ^{a}.- Nasal inhaler device that being specially envisaged as support for any aromatic and/or therapeutic product and having as purpose the personalized direct evaporation or atomization of this product, that is to say in the user's own nose, **characterized** because it is made up from a support body, equipped with means of securing to the user's nose, preferably to their nasal septum, a support body equipped in turn with means of support for at least one impregnator, soaker or porous capsule, made up of a spongy or porous body, as for example a small cotton bud, impregnated with the product in question or impregnable with this product.

2. ^{a}.- Nasal inhaler device, according to claim 1^{a}, **characterized** because the said support body is made up in a laminate piece, elastically deformable, preferably made from semi-rigid plastic or any other appropriate material, that adopts an omega configuration, so that by means of its central area it makes a securing clip to the nasal septum, while at its ends it makes pincers or supplementary fixings to embed or secure the respective infusers or porous capsules.
